# DEMANDE DE BREVET EUROPEEN

(11) **EP 0 938 850 A1**
(43) Date de publication de la demande: **01.09.1999**
(21) Numéro de dépôt: 99450002.3
(22) Date de dépôt: 15.01.1999
(51) Int. Cl.: A23L 1/304

(54) **Composition alimentaire ou médicamenteuse à base de fer et préparations la comprenant**

(30) Priorité: 16.01.1998 FR 9800624
(71) Demandeur: Investigations therapeutiques essais cliniques services société à Responsabilité Limitée, 33560 Sainte Eulalie (FR)
(72) Inventeur: Auzerie, Jack, 33560 Sainte-Eulalie (FR); Berbille, Hervé, 33000 Bordeaux (FR)
(74) Mandataire: Thébault, Jean-Louis

(57) **Abrégé**

L'objet de l'invention est une composition alimentaire ou médicamenteuse comprenant du fer ayant des caractéristiques améliorées d'assimilation par l'organisme humain en vue d'une lutte contre les carences en fer, caractérisée en ce qu'elle comprend en combinaison, au moins :
- un produit transporteur du fer et ayant au moins un effet bifidogène, et
- un sel de fer.

L'invention couvre aussi des préparations adaptées.

## Description

La présente invention concerne une composition alimentaire ou médicamenteuse à base de fer qui est particulièrement assimilable et dont la tolérance, y compris par les organismes infantiles est améliorée. La présente invention couvre aussi des préparations adaptées.

On constate actuellement que certaines catégories de personnes présentent des carences ou sub-carence en fer engendrées par des alimentations déséquilibrées mais aussi par des états physiques particuliers.

Les nourrissons, les femmes enceintes ou allaitantes, les adolescents ou les personnes âgées ont du mal à assimiler le fer.

Dans le cas des nourrissons, les anémies sont traitées à l'aide de préparations contenant des sels de fer simples comme le sulfate de fer, l'oxyde de fer ou le fumarate de fer associés à des compléments tels que la vitamine C et/ou des colorants ou parfums de type cacao, destinés à masquer le goût et la couleur.

Les effets secondaires relevés à la suite des traitements préconisants de telles préparations ont amené les prescripteurs à n'y recourir que pour les cas sérieux d'anémie. Ces préparations ne sont donc pas préconisées dans le cas de traitements préventifs ou d'entretien.

Les effets secondaires mentionnés se traduisent par des diarrhées, ou de la constipation ou encore une augmentation dans le tube digestif des bactéries Gram-pathogènes type E.Coli.

On note aussi que le fer a un effet oxydant, réaction de Fenton, qui pourrait selon certains auteurs être renforcé par la présence de vitamine C, en formant un radical ascorbyle.

On sait par ailleurs que ce type de préparation ne présente pas une très grande efficacité dans la mesure où la limite d'absorption par le corps humain n'excède pas 10 %. De ce fait, si les dosages sont augmentés, non seulement le taux d'absorption se trouve inchangé mais de plus, les effets secondaires associés s'en trouvent accentués par les quantités de fer non ingérées.

On ne peut qu'attribuer les effets secondaires au fer non assimilé.

Le but de l'invention est de proposer une composition qui comprend du fer et un composé qui améliore son assimilation. De ce fait, puisque les quantités sont diminuées de façon générale et les quantités non absorbées sont également minimisées, les effets secondaires se trouvent réduits d'autant. De plus, le composé qui favorise l'assimilation permet aussi d'améliorer la tolérance par une limitation des effets secondaires.

A cet effet, la présente composition alimentaire ou médicamenteuse comprenant du fer ayant des caractéristiques améliorées d'assimilation par l'organisme humain en vue d'une lutte contre les carences en fer, se caractérise en ce qu'elle comprend en combinaison, au moins :
- un produit transportant le fer et ayant au moins un effet bifidogène, et
- un sel de fer.

Ce produit transporteur du fer et à effet bifidogène est de la lactoferrine ou un prébiotique.

Selon une autre caractéristique, le produit ayant au moins un effet bifidogène est une combinaison de la lactoferrine et d'un prébiotique, par exemple un fructo-oligosaccharide.

Le sel de fer utilisé est avantageusement du chlorure ferrique.

La lactoferrine peut être saturée en fer.

L'invention est maintenant décrite ci-après sur la base de plusieurs exemples de préparation qui incluent toutes un composé choisi parmi les protéines, notamment la lactoferrine et/ou un prébiotique.

La lactoferrine est une protéine présente dans le lait des mammifères, notamment chez la femme et dans d'autres sources telles que l'oeuf.

Une composition selon l'invention, particulièrement efficace consiste à associer du fer sous forme de sel avec de la lactoferrine. Plus spécifiquement encore, le sel retenu est du chlorure ferrique.

L'invention couvre aussi des préparations particulières adaptées.

On constate que la lactoferrine a un effet antioxydant vis à vis du fer sous forme de sel et une effet anti-bactérien vis à vis des bactéries pathogènes.

Ces deux effets sont complétés par un troisième, la lactoferrine présente un effet bifidogène qui favorise l'implantation et le développement dans le tube digestif des Bifidobactéries symbiotiques qui à leur tour créent une barrière et gênent l'implantation et le développement des bactéries pathogènes.

On constate que cette composition permet de limiter de façon certaine les effets secondaires indésirables habituellement liés à la prise de fer.

Cette composition peut être complétée par association de molécules ou micro-organismes qui possèdent des propriétés similaires ou complémentaires. C'est par exemple le cas du lactose qui sert de substrat pour le développement des Bifidobactéries.

Ainsi, une composition particulièrement adaptée consiste à préparer une poudre soluble à reconstituer, un sirop, un comprimé, une capsule ou une gélule avec les dosages adaptés pour des prises de :
- 1µg à 10 g/jour de sel de fer
- 0.1 µg à 20 g/jour de lactoferrine

Une formule adaptée est indiquée ci-après :
Chlorure ferrique : 7 mg
Lactoferrine : 10 mg
support : qsp (quantité suffisante pour)
arôme : qsp

Une composition améliorée consiste à substituer à la lactoferrine, au moins un prébiotique qui favorise l'implantation et le développement d'un type de micro-organisme symbiotique et s'oppose à l'implantation des bactéries Gram-pathogènes. Le sel de fer est conservé.

On peut citer comme exemple de prébiotique un fructo-oligosaccharide à effet bifidogène qui favorise l'implantation des Bifidobactéries et qui s'oppose au développement d'E.Coli.

De plus, le métabolisme fermentaire des Bifidobactéries est réducteur, ce qui diminue le pouvoir oxydant du fer.

Cette composition peut faire l'objet de différentes formes galéniques en fonction des types d'ingestion, la voie orale étant préférée et dans ce cas, il convient d'adapter la forme à l'utilisateur final, nourrisson, enfant, adolescent ou adulte.

Ainsi, une composition particulièrement adaptée consiste à préparer une poudre soluble à reconstituer, un sirop, un comprimé, une capsule ou une gélule avec les dosages adaptés pour des prises de :
- 1µg à 10 g/jour de sel de fer
- 0,1µg à 20 g/jour de prébiotique

Une formule adaptée est indiquée ci-après :
chlorure ferrique : 7 mg
fructo-oligosaccharide: 1200 mg
support : qsp (quantité suffisante pour)
arôme : qsp

Une composition encore améliorée consiste à associer la lactoferrine et un prébiotique à un sel de fer. L'effet anti-bactérien de la lactoferrine est renforcé par le prébiotique ajouté mais simultanément, on obtient l'effet anti-oxydant de la lactoferrine qui manque aux prébiotiques. En effet, la lactoferrine se lie au fer libre, ce qui lui évite d'être oxydant.

L'absorption du fer est ainsi facilitée, sans augmenter inutilement les doses et en réduisant d'autant les effets secondaires.

Ainsi, une composition particulièrement adaptée consiste à préparer une poudre soluble à reconstituer, un sirop, un comprimé, une capsule ou une gélule avec les dosages adaptés pour des prises de :
- 1µg à 10 g/jour de sel de fer
- 0,1µg à 20 g/jour de lactoferrine
- 0,1µg à 20 g/jour de prébiotique

Une formule adaptée est indiquée ci-après :
chlorure ferrique : 7 mg
lactoferrine : 10 mg
fructo-oligosaccharide: 1200 mg
support : qsp (quantité suffisante pour)
arôme : qsp

## Revendications

1. Composition alimentaire ou médicamenteuse comprenant du fer ayant des caractéristiques améliorées d'assimilation par l'organisme humain en vue d'une lutte contre les carences en fer, caractérisée en ce qu'elle comprend en combinaison, au moins :
- un produit transporteur du fer et/ou ayant au moins un effet bifidogène, et
- un sel de fer.

2. Composition alimentaire ou médicamenteuse selon la revendication 1, caractérisée en ce que le produit transporteur du fer et ayant au moins un effet bifidogène est de la lactoferrine

3. Composition alimentaire ou médicamenteuse selon la revendication 1, caractérisée en ce que le produit ayant au moins un effet bifidogène est un prébiotique.

4. Composition alimentaire ou médicamenteuse selon la revendication 3, caractérisée en ce que le prébiotique est un fructo-oligosaccharide.

5. Composition alimentaire ou médicamenteuse selon la revendication 2, 3, caractérisée en ce que le produit ayant au moins un effet bifidogène est une combinaison de la lactoferrine et d'un prébiotique.

6. Composition alimentaire ou médicamenteuse selon les revendications 4 et 5, caractérisée en ce que le produit ayant au moins un effet bifidogène est une combinaison de la lactoferrine et d'un fructo-oligosaccharide.

7. Composition alimentaire ou médicamenteuse selon l'une quelconque des revendications précédentes, caractérisée en ce que le sel de fer est du chlorure ferrique.

8. Composition alimentaire ou médicamenteuse selon la revendication 7, caractérisée en ce qu'elle comprend les dosages suivants :
- 1 µg à 10 g/jour de sel de fer,
- 0,1 µg à 20 g/jour de lactoferrine et/ou
- 0,1 µg à 20 g/jour de fructo-oligosaccharide.

9. Préparation en poudre soluble à reconstituer, en sirop, en comprimé, en capsule ou en gélule ayant la composition alimentaire ou médicamenteuse selon la revendication 8, caractérisée en ce qu'elle comprend les dosages suivants :
- chlorure ferrique 7 mg
- lactoferrine 10 mg
- support : qsp
- arôme : qsp

10. Préparation en poudre soluble à reconstituer, en sirop, en comprimé, en capsule ou en gélule ayant la composition alimentaire ou médicamenteuse selon la revendication 8, caractérisée en ce qu'elle comprend les dosages suivants :
- chlorure ferrique 7 mg
- fructo-oligosaccharide 1200 mg
- support : qsp
- arôme : qsp

11. Préparation en poudre soluble à reconstituer, en sirop, en comprimé, en capsule ou en gélule ayant la composition alimentaire ou médicamenteuse selon la revendication 8, caractérisée en ce qu'elle comprend les dosages suivants :
- Chlorure ferrique 7 mg
- fructo-oligosaccharide 1200 mg
- lactoferrine 10 mg
- support : qsp
- arôme : qsp
